Europäisches Patentamt

European Patent Office

Office européen des brevets

(11) Numéro de publication: **0 086 152 B1**

(12) **FASCICULE DE BREVET EUROPEEN**

(45) Date de publication du fascicule du brevet:
21.05.86

(21) Numéro de dépôt: 83400233.9

(22) Date de dépôt: 04.02.83

(51) Int. Cl.⁴: **A 61 K 45/06**, A 61 K 39/395 //
(A61K39/395, 31:35)

(54) Médicaments comportant en tant qu'association au moins une immunotoxine et au moins un ionophore carboxylique monovalent.

(30) Priorité: 09.02.82 FR 8202091

(43) Date de publication de la demande:
17.08.83 Bulletin 83/33

(45) Mention de la délivrance du brevet:
21.05.86 Bulletin 86/21

(84) Etats contractants désignés:
AT BE CH DE FR GB IT LI LU NL SE

(56) Documents cités:
BIOLOGICAL ABSTRACTS, vol. 72, no. 12, 1981, no. 84782, Philadelphia, Pennsylvania, US, S.R. BISWAIJT et al.: "Enhancement of cytetoxicities of ricin and Pseudomonas toxin in Chinese hamster ovary cells by nigericin"
BIOLOGICAL ABSTRACTS, vol. 72, no. 12, 1981, no. 84783, Philadelphia, Pennsylvania, US, R. BISWAIJT et al.: "Enhanced internalization of ricin in nigericin-pretreated Chinese hamster ovary cells"
NATURE, vol. 290, 12 mars 1981, pages 145-146, Macmillan Journals Ltd., Chesham, GB, H.E. BLYTHMAN et al.: "Immunotoxins: hybrid molecules of monoclonal antibodies and a toxin subunit specifically kill tumour cells"

(73) Titulaire: SANOFI, société anonyme, 40, Avenue George V, F-75008 Paris (FR)

(72) Inventeur: Jansen, Franz, Chemin des Sesquets, F-34160 Assas (FR)
Inventeur: Gros, Pierre, 18 rue des Muriers, F-34100 Montpellier (FR)

(74) Mandataire: Gillard, Marie-Louise et al, Cabinet Beau de Loménie 55, Rue d'Amsterdam, F-75008 Paris (FR)

LIBER, STOCKHOLM 1986

## Description

La présente invention concerne de nouveaux médicaments comportant en association au moins une immunotoxine et au moins un ionophore carboxylique monovalent.

Dans les demandes de brevets français antérieurs portant notamment les numéros 78.27 838 (FR-A-2437213), 79.24 655 (FR-A-2466252), 81.07 596 (EP-A-63988) et 81.21 836 (EP-A-80401), on a décrit la préparation de produits anticancéreux dits conjugués obtenus par couplage, par liaison covalente, de la chaîne A de la ricine avec des anticorps ou fragments d'anticorps dirigés contre un antigène porté par une cellule à détruire. Les produits de ce type sont désignés dans la présente demande sous le nom générique d'immunotoxines.

Dans la demande française 81 21 836 (EP-A-80401) on a décrit en outre la propriété des ions ammonium (sous la forme d'un quelconque de leurs sels et en particulier le chlorure) de potentialiser de façon efficace l'action cytotoxique de ces immunotoxines.

La propriété des sels d'ammonium de potentialiser l'activité cytotoxique sélective des immunotoxines présente de nombreux avantages dans deux types de situation.

a) Chaque fois qu'une immunotoxine est utilisée en tant qu'agent cytotoxique sélectif in vitro pour détruire les cellules cibles.

Dans le cas des applications thérapeutiques cette situation se rencontre notamment lorsque l'immunotoxine est utilisée comme agent cytotoxique dans le traitement de la moëlle osseuse de patients leucémiques à qui la moëlle osseuse ainsi traitée sera ultérieurement transplantée, ainsi que cela a été décrit dans la demande de brevet déposée en France sous le n° 81 21836 ( =EP-A-80401) par la demanderesse.

b) Lorsque l'immunotoxine est utilisée in vivo en tant qu'agent thérapeutique chez l'homme chaque fois qu'il est possible d'administrer préventivement, simultanément ou ultérieurement au malade un sel d'ammonium assurant la potentialisation de l'effet de l'immunotoxine ainsi que cela a été décrit dans la demande de brevet indiquée ci-dessus.

Toutefois, dans ce dernier cas d'utilisation in vivo de l'immunotoxine l'emploi également in vivo d'un sel d'ammonium afin de bénéficier de l'effet potentialisateur connaît certaines limitations inhérentes à le toxicité propre des ions ammonium et au fait qu'il est relativement malaisé de maintenir de façon durable une concentration suffisante en ions ammonium dans les liquides biologiques du malade.

La présente invention a pour objet la préparation de médicaments cytotoxiques puissants utilisant la potentialisation des effets cytotoxiques sélectifs des immunotoxines décrites dans les demandes antérieures mentionnées précédemment.

Après que de nombreuses substances ont été étudiées sans succès, il a été trouvé que les ionophores carboxyliques monovalents choisi parmi la monensine, la nigéricine, la grisorixine et le lasalocide représentaient un groupe de substances particulièrement intéressantes pour potentialiser l'effet cytotoxique des immunotoxines.

Par ionophores carboxyliques on désigne des substances - naturelles isolées à partir de souches de Streptomyces qui comportent un squelette linéaire hydrocarboné dans lequel sont inclus des hétérocycles oxygénés. A une extrémité de la chaîne il existe toujours une fonction acide carboxylique tandis qu'à l'autre extrémité se trouvent une ou plusieurs fonctions alcools [voir B. C. Pressman : Annual Review of Biochemistry 45 501-530 (1976)].

Ces produits présentent la propriété de former des complexes liposolubles avec de nombreux cations permettant à ces ions de franchir les barrières lipidiques.

Ces ionophores carboxyliques monovalents possèdent diverses propriétés biologiques et notamment des propriétés antibiotiques et antiparasitaires. Aussi, par exemple la monensine est utilisée comme adjuvant dans l'alimentation du bétail et comme agent coccidiostatique chez les volailles.

Il a été indiqué également [B. Ray et H. C. Wu, Molecular and Cellular Biology 1, 552-559 (1981)] que la monensine et la nigericine augmentent la cytotoxicité de la ricine vis-à-vis de certaines lignées cellulaires. Cette augmentation est toutefois modeste de l'ordre d'un facteur 10.

Dans la présente invention, il a été trouvé que de façon surprenante, les ionophores carboxyliques monovalents utilisés à des doses où ils ne présentent eux-mêmes aucune cytotoxicité propre pour les lignées étudiées potentialisent de façon extrêmement importante (facteurs de 7 000 à 55 000) la cytotoxicité spécifique des immunotoxines.

L'exemple suivant non limitatif permet de mieux comprendre la portée de l'invention.

### Exemple

Cet exemple démontre la potentialisation de la cytotoxicité sélective de l'immunotoxine anti-T-65 (telle que décrite dans la demande n° 81.21836 de la demanderes se) vis-à-vis des cellules lymphoblastoïdes T humaines de la lignée CEM portant l'antigène T-65.

Dans ces expériences, la cytotoxicité a été évaluée par la mesure de l'incorporation de $^{14}$C-leucine par les cellules après 24 h d'incubation à 37 °C en présence de quantités connues de l'immunotoxine étudiée, ou de substances cytotoxiques de référence, en absence ou en présence des ionophores à tester.

1 - Potentialisation de l'effet cytotoxique par les ionophores.

Les résultats de ces expériences sont présentés sous la forne de courbes dose/effet présentant en ordonnée l'effet cytotoxique évalué comme indiqué ci-dessus par l'incorporation du traceur, calculée en % de la valeur obtenue sur les cellules témoins sans substance cytotoxique, et en abscisse les concentrations molaires en sous-unité toxique des substances cytotoxiques étudiées. La monensine et la grisorixine ont été testées à la concentration de 50 nanomolaire la nigéricine à la concentration de 10 nanomolaire et le lasalocide à la concentration de 1 micromolaire. Il a été préalablement vérifié que ces substances ne sont pas spontanément cytotoxiques pour les cellules employées, aux concentratioms indiquées.

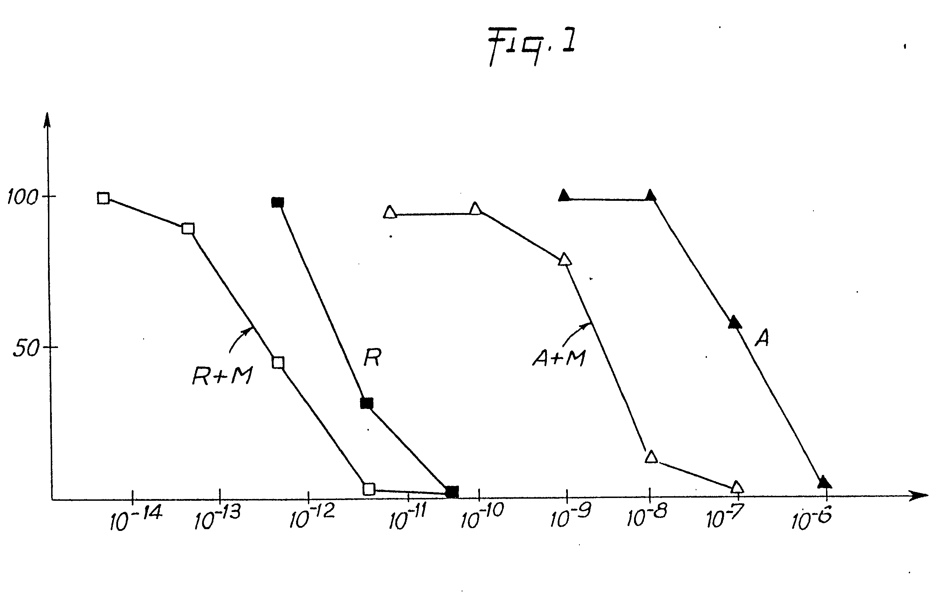

La figure 1 montre l'effet de la monensine sur la cytotoxicité propre de la ricine (R) et de sa chaîne (A) isolée prises comme substances de référence. Les valeurs des concentrations molaires (CI 50) correspondant à 50% d'inhibition d'incorporation du traceur sont indiquées dams le tableau I. Les quatre courbes obtenues sont relatives respectivement à : la chaîne A de la ricime (A) un mélange de la chaîme A de la ricine et de monensine $(A)+M$ la ricine (R) et un mélange de ricine et de monensine $(R)+M$.

### TABLEAU I

| Substances testées | Avec monensine 50 nM | Sans monensine |
|---|---|---|
| Ricine (R) | $4.10^{-13}$ M | $2,8.10^{-12}$ M |
| Chaîne A (A) | $2,7.10^{-9}$ M | $1,3.10^{-7}$ M |

Ces résultats démontrent un certain effet potentialisateur de la monensine sur la cytotoxicité de la ricine (facteur de potentialisatiom de 7) et de la chaîne A (facteur de potentialisation de 47).

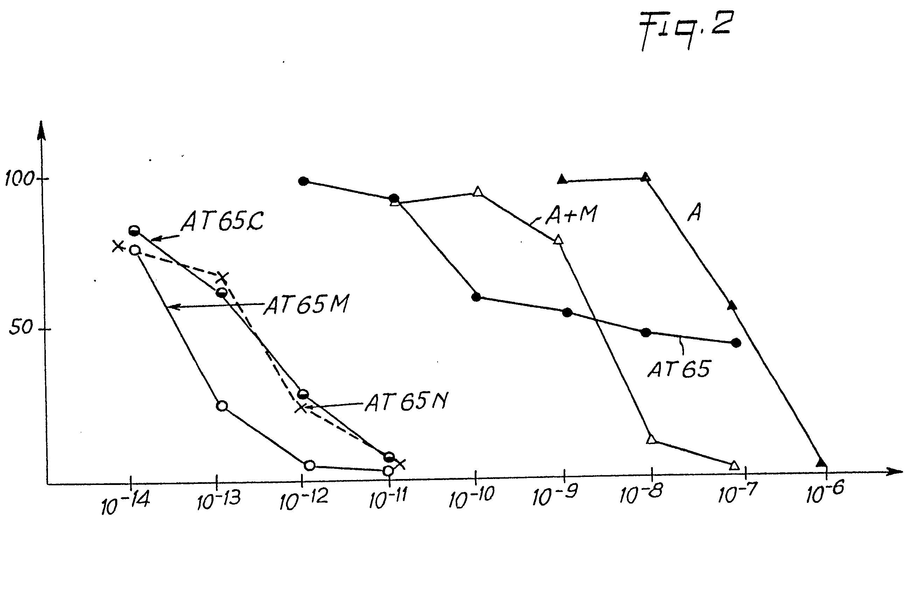

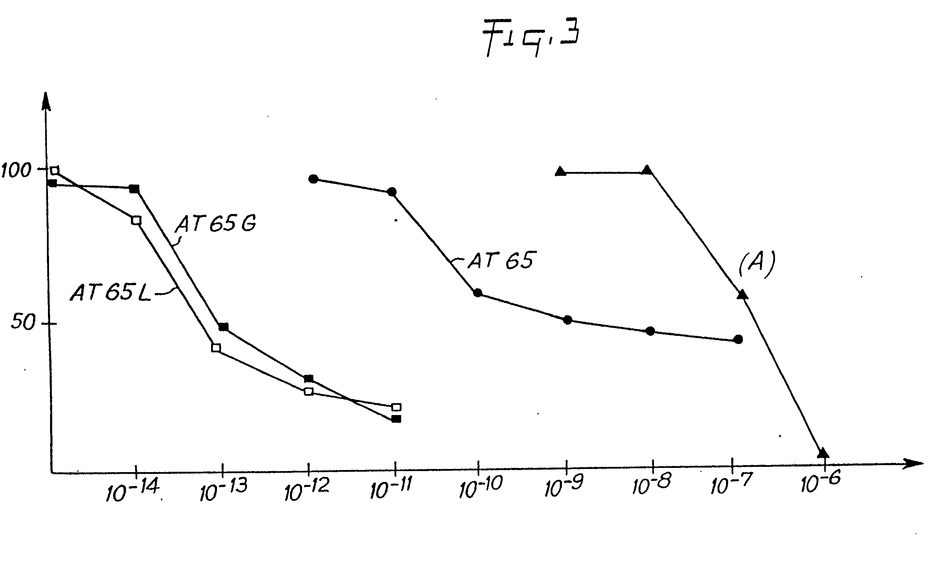

Les figures 2 et 3 momtrent l'effet potentialisateur comparé de l'ion $NH_4^+$ (10 mM) de la monensine (50 mM) de la nigéricine (10 nM), de la grisorixine (50 mM) et du lasalocide (1µM) sur la cytotoxicité de l'immumotoxine anti-T-65 vis-à-vis des cellules de la lignée CEM. Les valeurs des concentrations molaires (CI 50) correspondant à 50% d'inhibition d'incorporation du traceur sont rappelées dans le tableau II.

Sur ces figures les diverses courbes s e rapportent respectivement aux résultats obtenus avec : la chaime A de la ricine (A) la chaîne A de la ricime mé langée avec la monensine $(A)+(M)$ le conjugué anti-T-65 (AT65) et le conjugué AT65 additionné de nigéricine (AT65N), monensine (AT65M) chlorure d'ammonium (AT65C), grisorixine (AT650) et lasalocide (AT65L).

Ces résultats montrent que l'effet potentialisateur de la nigéricine est très vois in de celui de l'ion ammonium et de l'ordre d'um facteur de 7000 tandis. que celui de la monensine est nettement plus élevé de l'ordre de 55000. La grisorixine et le lasalocide occupent une position intermédiaire avec des effets potentialisateurs de l'ordre de 27000 et 36000 respectivement. Ces facteurs sont considérablement plus élevés que ceux observés avec la ricine ou la chaîne A isolée. Il faut aussi remarquer que, en présence de monensine notamment, l'immunotoxine anti-T-65 est un agent cytotoxique près de 10 fois plus puissant que la ricine ellemême, tout en présentant (contrairement à la ricine) un remarquable facteur de sélectivité pour les seules cellules-cibles.

### TABLEAU II

| Substances potentialisatrices testées | CI 50 |
|---|---|
| Aucune | $2,2.10^{-9}$ M |
| $NH_4^+$ 10 mM | $3.10^{-13}$ M |
| Monensine 50 nM | $4.10^{-14}$ M |
| Nigéricine 10 nM | $3.10^{-13}$ M |
| Grisorixine 50 nM | $8.10^{-14}$ M |
| Lasalocide 1 µM | $6.10^{-14}$ M |

2 - Accélération des cinétiques de cytotoxicité par les ionophores.

L'effet des ionophores ne se limite pas à accroître d'une façon considérable l'activité cytotoxique des immunotoxines. Ces substances permettent aussi d'accélérer de façon très importante la cinétique de cytotoxicité des immunotoxines comme le montrent les expériences suivantes :

Dans ces expériences, on a mesuré comme précédemment l'incorporation de traceur radioactif dans les cellules mais cette fois en fonction du temps d'incubation des cellules avec l'immunotoxine, en absence et en présence de monensine 50 nM comme potentialisateur. Cette expérience a été réalisée sur deux modèles cellulaires en utilisant les immunotoxines de spécificités correspondantes à savoir:.

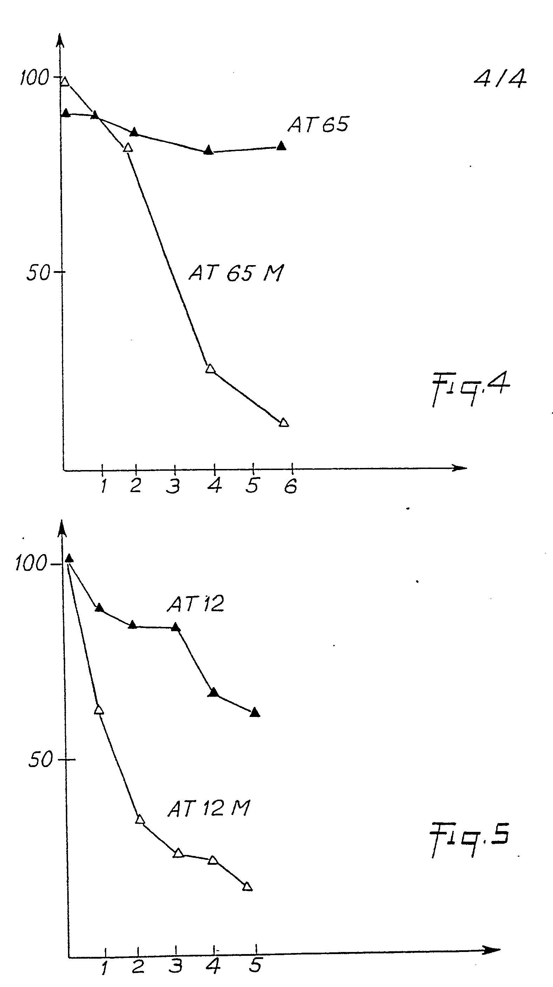

. La lignée lymphoblastoïde humaine CEM avec l'immunotoxine anti- T-65 à la concentration de 50 nM. Les résultats sont présentés dans la figure 4. Sur cette figure les courbes se rapportent respectivement à l'anti-T-65 (AT65) et au mélange d'anti-T-65 et de monensine (AT65M).

. La lignée de lymphome de souris WEHI 7 avec l'immunotoxine anti-Thy 1,2 (telle que décrite dans la demande n° 79 24655 de la demanderesse la concentration de 50 mM. Les résultats sont présentés dans la figure 5. Sur cette figure les courbes se rapportent respectivement au conjugu anti-Thy 1.2 (ATI2) et au mélange de ce conjugué avec la monensine (ATI2. M).

Pour la lignée CEM il apparaît que en l'absence de potentialisation, la cinétique de cytotoxicité est très lente comme le montre la courbe a. D'autres expériences dans les mêmes conditions ont montré que le temps nécessaire à obtenir 50%

de réduction de l'incorporation du traceur était de l'ordre de 20 heures. Par contre, en présence de monensine une accélération spectaculaire de la cinétique se manifeste (courbe b) puisque le temps nécessaire à obtenir 50% d'inhibition d'incorporation est alors de l'ordre de 3 heures seulement.

Pour la lignée WEHI 7 les résultats sont analogues puisque les temps nécessaires pour obtenir 50% d'inhibition d'incorporation sont respectivement, en absence et en présence de nonensine : 10 heures et 1,5 heure.

Un tel effet d'accélération est de la plus haute importance pour toutes applicatione des immunotoxines et en particulier pour les applications thérapeutiques in vivo car la rapidité d'action du médicament est toujours un facteur très favorable à l'efficacité du traitement.

Par ailleurs, nous avons étudié la toxicité des ionophores carboxyliques monovalents soit seule soit en association avec la chaîne A de Ricine.

D'après J.W. Westley (Advances in Applied Microbiology, 22, 177, (1977)], les doses létales 50% (DL 50) chez la souris par voie intra-péritonéale sont respectivement de : 16,8 mg/kg pour la monensine, 65 mg/kg pour la nigéricine, 15 mg/kg pour la grisorixine et 64 mg/kg pour le lasalocide.

Ces résultats ont été confirmés par nos propres expériences. Ainsi, la toxicité aiguë de la monensine en administration unique par voie intra-péritonéale chez la souris Charles River France CDI est caractérisée par une dose létale 50% de 340 microgrammes par souris, soit 17 mg/kg de poids corporel.

En outre comme il a été montré ci-dessus que la monensine potentialise légèrement l'activité cytotoxique de la chaîne A de ricine sur des cellules en culture il était important de vérifier sur ce système modèle de cytotoxicité non spécifique, quel était l'impact toxicologique global de cette propriété sur l'animal entier. Pour cela nous avons détermimé la dose létale 50% de la chaîne A de ricine administrée par voie intrapéritonéale chez la souris Charles River France CDI en l'absence ou avec coadministration de 10 microgrammes ou 20 microgrammes de monensine par souris, par voie intrapéritonéale.

Les valeurs trouvées sont les suivantes :
- DL 50 Chaîne A seule 511 microgrammes par souris
- DL 50 Chaîne A + 10 μg monensine 363 " "
- DL 50 Chaîne A + 20 μg monensine 203 " "

Ces résultats montrent une légère augmentation de toxicité de la Chaîne A loraqu'elle est administrée en même temps que la monensine. Cet accroissement de toxicité par un facteur de seulement 2,5 (pour la plus forte dose de monensine expérimentée) ne constitue pas une limitation à l'emploi in vivo des ionophores comptetenu des effets potentialisateur et accélérateur très important vis-à-vis de la cytotoxicité spécifique des immunotoxines comme cela a été montré ci-dessus.

On peut donc utiliser en tant que médicaments en thérapeutique humaine l'association constituée par une immunotoxine et un ionophore carboxylique monovalent. Ils peuvent être utilisés pour le traitement des affections, cancéreuses ou non, qui seraient sensibles à l'anticorps utilisé pour la préparation de l'immunotoxine.

Visant à éliminer la totalité des cellules cancéreuses, le traitement devra être effectué avec une dose suffisante d'immunotoxine associée avec une quantité d'ionophore carboxylique monovalent pouvant varier de 1 à ICO mg à chaque administration d'immunotoxine. La durée du traitement devra être déterminée dans chaque cas en fonction du sujet et de la nature de l'affection à traiter.

Les nouveaux médicaments selon l'invention sont conditionnés pour être utilisés par voie injectable et de préférence par voie intraveineuse.

De préférence les constituants de l'association seront conservés séparément et mélangés, seulement au moment de l'emploi, dans la seringue ou le solvant de perfusion.

**Revendications** pour les états contractants BE CH DE FR GB IT LI LU NL SE

1. Médicaments caractérisés en ce qu'ils comportent, en association, au moins une immunotoxine obtenue par couplage, par liaison covalente, de la chaîne A de la ricine avec des anticorps ou fragments d'anticorps dirigés contre un antigène porté par une cellule à detruire et au moins un ionophore carboxylique monovalent choisi parmi la monensine, la nigéricine, la grisorixine et le lasalocide.

2. Médicaments selon la revendication 1, caractérisés en ce que l'immunotoxine est l'immonotoxine anti-T-65.

3. Médicamente selon la revendication 1, caractérisés en ce que l'immunotoxine est l'immunotoxine anti-Thy 1. 2.

4. Médicament selon l'une quelconque des revendications 1 à 3, caractérisé en ce qu'il est conditionné de façon à être utilisé par voie injectable, de préférence par voie intraveineuse.

5. Utilisation d'au moins un ionophore carboxylique monovalent choisi parmi la monensine, la nigéricine, la grisorixine et le lasalocide pour la potentialisation de l'effet cytotoxique des immunotoxines obtenues par couplage par liaison covalente de la chaîne A de la ricine avec des anticorps ou fragments d'anticorps dirigés contre un antigène porté par une cellule à détruire.

**Revendications** pour l'etat contractant : AT.

1. Utilisation d'au moins un ionophore carboxylique monovalent choisi parmi la monensine, la nigericine, la grisorixine et le lasalocide pour la potentialisation de l'effet cytotoxique des immunotoxines obtenues par couplage par liaison covalente de la chaîne A de la ricine avec des anticorps ou fragments d'anticorps dirigés contre un antigene porté par une cellule à détruire.

2. Utilisation selon la revendication 1, caractérisée en ce que l'immunotoxine est l'immunotoxine anti-T.65.

3. Utilisation selon la revendication 1, caractérisée en ce que l'immunotoxine est l'immunotoxine anti-Thy 1.2.

4. Utilisation d'au moins un ionophore carboxylique monovalent choisi parmi la monensine, la nigericine, la grisorixine et le lasalocide pour la préparation d'un médicament comportant en association au moins une immunotoxine obtenue par couplage par liaison covalente de la chaîne A de la ricine avec des anticorps ou fragments d'anticorps dirigés contre un antigène porté par une cellule à détruire et au moins un ionophore carboxylique monovalent ledit medicament étant conditionné de façon à être utilisé par voie injectable, de préférence par voie intraveineuse.

**Patentansprüche** für die Vertragsstaaten BE, CH, DE, FR, GB, IT, LI, LU, NI, SE:

1. Medikamente, dadurch gekennzeichnet, daß sie in Kombination mindestens ein Antitoxin, das durch Kopplung mittels kovalenter Bindung der Kette A des Ricins mit Antikörpern oder Fragmenten von Antikörpern, die gegen ein von einer zu zerstörenden Zelle getragenes Antigen gerichtet sind, erhalten wurde,und mindestens einen monovalenten carboxylischen Ionophor, ausgewählt aus Monensin, Nigericin, Grisorixin und Lasalocid, enthalten.

2. Medikamente nach Anspruch 1, dadurch gekennzeichnet, daß das Antitoxin Antitoxin anti-T-65 ist.

3. Medikamente nach Anspruch 1, dadurch gekennzeichnet, daß das Antitoxin Antitoxin anti-Thy 1.2 ist.

4. Medikament nach einem der Ansprüche 1 bis 3, dadurch gekennzeichnet, daß es zur Verwendung auf Injektionsweg, vorzugsweise auf intravenösem Weg, konditioniert ist.

5. Verwendung von mindestens einem monovalenten carboxylischen Ionophor, ausgewählt aus Monensin, Nigericin, Grisorixin und Lasalocid zur Potenzierung der cytotoxischen Wirkung von Antitoxinen, die durch Kopplung mittels kovalenter Bindung der Kette A des Ricins mit Antikörpern oder Fragmenten von Antikörpern, die gegen ein von einer zu zerstörenden Zelle getragenes Antigen gerichtet sind, erhalten wurden.

**Patentansprüche** für den Vertragsstaat AT:

1. Verwendung mindestens eines monovalenten carboxylischen Ionophors, ausgewählt aus Monensin, Nigericin, Grisorixin und Lasalocid, zur Potenzierung der cytotoxischen Wirkung von Antitoxinen,die durch Kopplung mittels kovalenter Bindung der Kette A des Ricins mit Antikorpern oder Fragmenten von Antikörpern, die gegen ein von einer zu zerstörenden Zelle getragenes Antigen gerichtet sind, erhalten wurden.

2. Verwendung nach Anspruch 1, dadurch gekennzeichnet, daß das Antitoxin Antitoxin anti-T.65 ist.

3. Verwendung nach Anspruch 1, dadurch gekennzeichnet, daß das Antitoxin Antitoxin anti-Thy 1.2 ist.

4. Verwendung mindestens eines monovalenten carboxylischen Ionophors, ausgewählt aus Monensin, Nigericin, Grisorixin und Lasalocid, zur Herstellung eines Medikaments, welches in Kombination mindestens ein Antitoxin, das durch Kopplung mittels kovalenter Bindung der Kette A des Ricins mit Antikörpern oder Fragmenten von Antikörpern, die gegen ein von einer zu zerstörenden Zelle getragenes Antigen gerichtet sind, erhalten wurde, und mindestens einen monovalenten carboxylischen Ionophor enthält, wobei das Medikament zur Verwendung auf Injektionsweg, vorzugsweise auf intravenösem Weg, konditioniert ist.

**Claims** for the contracting States: BE, CH, DE, FR, GB, IT, LI, LU, NL, SE

1. Drugs characterized in that they comprise, in association, at least one immunotoxin obtained by antibodies or antibodies fragments directed against an antigen carried by a cell to be destroyed, and at least one monovalent carboxylic ionophora selected from monensin, nigericin, grisorixin and lasalocide.

2. Drugs according to claim 1, characterized in that the immunotoxin is anti-T-65 immunotoxin.

3. Drugs according to claim 1, characterized in that the immunotoxin is anti-Thy 1.2. immunotoxin.

4. A drug according to any one of claims 1 to 3, characterized in that it is packaged so as to be used by the injectable route, preferably by the intravenous route.

5. Use of at least one monovalent carboxylic ionophora selected from monensin, nigericin, grisorixin and lasalocide for potentializing the cytotoxic effect of immunotoxins obtained by coupling, by covalent bond, the A chain of ricin with antibodies or antibodies fragments directed against an antigen carried by a cell to be destroyed.

**Claims** for the contracting state: AT

1. Use of at least one monovalent carboxylic ionophora selected from among monensin, nigericin, grisorixin and lasalocide for the potentiation of the cytotoxic effect of immunotoxins obtained by coupling, by covalent bond, the A chain of ricin with antibodies or antibodies fragments directed against an antigen borne by a cell to be destroyed.

2. Use according to claim 1, characterized in that the immunotoxin is anti-T65 immunotoxin.

3. Use according to claim 1, characterized in that the immunotoxin is anti-Thy 1.2.

4. Use of at least one monovalent carboxylic ionophora selected from among monensin, nigericin, grisorixin and lasalocide for the preparation of a drug comprising in association at least one immunotoxin obtained by coupling, by covalent bond, the A chain of ricin with antibodies or antibodies fragments directed against an antibody borne by a cell to be destroyed and at least one monovalent carboxylic ionophora, said drug being packaged so as to be used by the injectable route, preferably by the intravenous route.

Fig.1

Fig. 2

0 086 152

Fig. 3

4/4

Fig.4

Fig.5